# EUROPÄISCHE PATENTSCHRIFT

(11) **EP 0 675 881 B1**
(45) Veröffentlichungstag und Bekanntmachung des Hinweises auf die Patenterteilung: **19.03.1997**
(21) Anmeldenummer: 94902771.8
(22) Anmeldetag: 15.12.1993
(51) Int. Cl.: C07D 251/08, C07D 253/08, A61K 31/53

(54) **Verwendung von Benzotriazinen als PLA2-Inhibitoren und neue Benzotriazine**
Use of benzotriazines as PLA2-Inhibitors and new benzotriazines
Utilisation de benzotriazines comme inhibiteurs de la PLA-2 et nouvelles benzotriazines

(30) Priorität: 24.12.1992 DE 4244009
(43) Veröffentlichungstag der Anmeldung: 11.10.1995
(73) Patentinhaber: BOEHRINGER MANNHEIM GMBH, 68298 Mannheim (DE)
(72) Erfinder: FRIEBE, Walter-Gunar, D-68165 Mannheim (DE); SCHEUER, Werner, D-83646 Bad Tölz (DE); TIBES, Ulrich, D-60599 Krankfurt (DE)
(86) Internationale Anmeldenummer: EP9303542
(87) Internationale Veröffentlichungsnummer: WO9414781

(56) Entgegenhaltungen:
- FR-M- 7 933
- US-A- 2 911 406
- US-A- 2 966 487
- US-A- 3 079 390
- JOURNAL OF MEDICINAL CHEMISTRY. Bd. 11 , 1968 , WASHINGTON US Seiten 946 - 949 J. KENNETH HORNER ET AL. 'Analogs of 3-amino-7-chloro-1,2,4-benzotriazine 1-oxide as antimalarial agents'

## Beschreibung

Gegenstand der vorliegenden Erfindung ist die Verwendung von Benzotriazinen als PLA₂-Inhibitoren, neue Benzotriazinderivate, Verfahren zu deren Herstellung und Arzneimittel. die diese Verbindungen enthalten.

Die Erfindung betrifft die Verwendung von Benzotriazinderivaten der allgemeinen Formel I 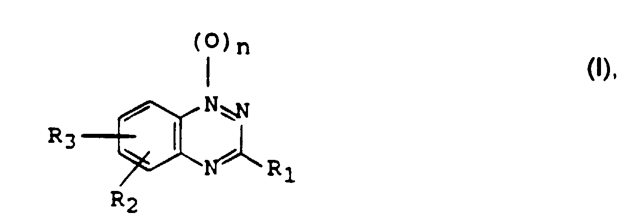 zur Herstellung von Arzneimitteln mit PLA₂-inhibierender Wirkung,
wobei
- R₁: eine Amino-, oder eine Hydroxygruppe,
- R₂ und R₃,: die gleich oder verschieden sind, jeweils Wasserstoff, Halogen, C₁-C₆-Alkyl, C₁-C₆-Alkoxy, Cyano, Carboxy, Cyano-C₁-C₆-alkyl, Carboxy-C₁C₆-alkyl, Hydroxy, Carboxy-C₁-C₆-alkoxy, C₁-C₆-Alkoxycarbonyl-C₁-C₆-alkoxy oder Aminocarbonyl-C₁-C₆-alkoxy, C₁-C₆-Alkylaminocarbonyl-C₁-C₆-alkoxy- oder Di-C₁-C₆-Alkylaminocarbonyl-C₁-C₆-alkoxy
und
- n: die Zahl 0 oder 1 bedeutet,
deren Tautomere sowie Salze mit nichttoxischen Säuren oder Basen.

Verbindungen der Formel I sind z. T. bekannt. So sind z.B. in J. Amer. Chem. Soc. 76, 3551 (1954) Verbindungen der Formel I mit R₂ = Cl oder H und R₁ = OH oder NH₂, R₃ = H und n = 0 oder 1 als Antimalariamittel beschrieben. Weitere Verbindungen mit n= 1 und R₁ = NH₂ sind aus J. Chem. Soc. (B) 1970, 911 bekannt. Sie werden in diesen Dokumenten als Farbstoffe, Radiosensitizer, Bactericida, Insecticida, Acaricida, cyctotoxische Mittel und Antimalariamittel beschrieben. Eine antientzündliche Wirkung ist nicht aufgeführt.

Die Verbindungen der Formel I weisen wertvolle pharmakologische Eigenschaften auf, insbesondere können sie die Aktivität von Phospholipasen hemmen. Sie eignen sich daher zur Behandlung akuter und chronischer, allergischer, nichtallergischer und traumatischer entzündlicher Erkrankungen, wie beispielsweise rheumatische Arthritis, Osteoarthritis, Ulcerative Colitis, akute Pankreatitis, Kontaktdermatitis, entzündliche und allergische Atemwegserkrankungen, septischer Schock, allergischer Schock, Serumkrankheit, Autoimmunerkrankungen, graft-versus-host- Reaktionen, host-versus-graft-Erkrankungen, ischämische oder thrombotische Erkrankungen, beispielsweise Coronarinfarkt oder Cerebralinfarkt.

Die "Alkylteile" in den bei R² und R³ genannten aliphatischen Gruppen können geradkettig oder verzweigt sein. Bevorzugte Reste sind der Methyl-, Ethyl-, n-Propyl-, Isopropyl-, n-Butyl-, Isobutyl-, tert.-Butyl-, n-Pentyl- und 3-Pentylrest. Als C₁-C₆-Alkoxygruppe kommt in diesem Sinne beispielsweise die Methoxy-, Ethoxy-, Propoxy- oder Butoxygruppe in Frage.

Halogenatome sind insbesondere Fluor, Chlor und Brom.

Außer den in den Beispielen genannten Verbindungen sind Gegenstand der Erfindung insbesondere alle Substanzen, die jede mögliche Kombination der in den Beispielen genannten Substituenten aufweisen.

Gegenstand der Erfindung sind auch neue Verbindungen der Formel I a 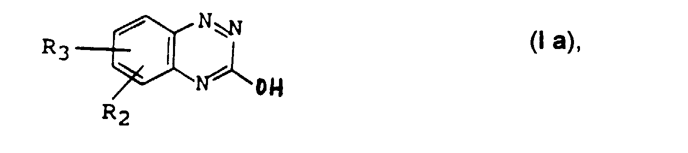 in der
- R₂: C₁-C₆-Alkyl, C₁-C₆-Alkoxy, Cyano, Carboxy, Cyano-C₁-C₆-alkyl, Carboxy-C₁-C₆-alkyl, Hydroxy, Carboxy-C₁-C₆-alkoxy, C₁-C₆-Alkoxycarbonyl-C₁-C₆-alkoxy, Aminocarbonyl-C₁-C₆-alkoxy, C₁-C₆-Alkylaminocarbonyl-C₁-C₆-alkoxy oder Di-C₁-C₆-Alkylaminocarbonyl-C₁-C₆-alkoxy und
- R₃: Wasserstoff oder C₁-C₆-Alkyl bedeuten,
deren Tautomere sowie Salze mit nichttoxischen Säuren oder Basen.

Die Verbindungen der Formel I bzw. I a eignen sich insbesondere zur Herstellung von Arzneimitteln zur Behandlung akuter oder chronischer Erkrankungen, insbesondere solcher Erkrankungen mit entzündlicher, immunologischer, allergischer, nichtallergischer oder traumatischer Genese. Da die Phospholipase A₂ (PLA₂) ein Schlüsselenzym bei der Entstehung dieser Erkrankungen ist, werden durch Hemmung dieses Enzyms derartige Erkrankungen wirkungsvoll behandelt. Es wurde nun überraschenderweise gefunden, daß die Verbindungen der Formel I bzw. I a die Phospholipase A₂ inhibieren, und somit aufgrund dieses neuen Wirkprofils bei der Behandlung von Erkrankungen eingesetzt werden können, bei denen die Hemmung der Phospholipase A₂ von klinischer, pathologischer oder symptomatischer Relevanz ist, insbesondere bei entzündlichen Erkrankungen.

Bei den Verbindungen der Formel I bedeutet R¹, vorzugsweise eine Amino- oder Hydroxygruppe.

Die Substituenten R² und R³ können im Fall der Verbindungen der Formel I oder I a unabhängig voneinander an 5-, 6-, 7- oder 8-Stellung des Benztriazolsystems stehen. Bevorzugt kommen am Phenylring monosubstituierte Derivate in Frage (R³ = Wasserstoff und R² kein Wasserstoff). Im Fall von disubstituierten Derivaten (weder R² noch R³ Wasserstoff) stehen die Substituenten R² und R³ vorzugsweise in 6-und 7-Stellung des Benztriazolsystems.

Bevorzugte Reste für R² sind insbesondere die folgenden Substituenten: Chlor, Methyl, Ethyl, Isopropyl, t.-Butyl, Methoxy, 1,1-Dimethyl-cyanomethyl, Hydroxy, Carboxy-methoxy. R³ bedeutet vorzugsweise Wasserstoff oder die Methylgruppe.

Das erfindungsgemäße Verfahren zur Herstellung der Verbindungen der Formel I bzw. I a ist dadurch gekennzeichnet, daß man in an sich bekannter Weise eine Verbindung der allgemeinen Formel II 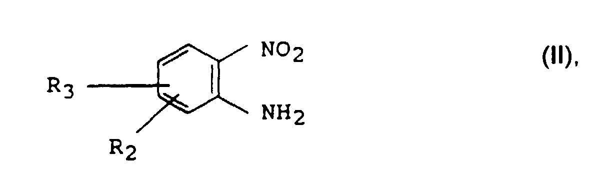 in welcher R₂ und R₃ die obengenannten Bedeutung haben, mit Cyanamid umsetzt und unter basischen Bedingungen cyclisiert, und anschließend das erhaltene Produkt durch Reduktion und anschließende Diazotierung und Hydrolyse in eine Verbindung der Formel I, in welcher R₂ und R₃ die obengenannte Bedeutung haben, überführt, sowie gegebenenfalls durch Neutralisation mit nichttoxischen Säuren oder Basen in ein Salz umwandelt.

Die Umsetzung von Verbindungen der Formel II mit Cyanamid erfolgt zweckmäßig in wässrigem Medium unter Protonenkatalyse und Erwärmung, beispielsweise in konz. Salzsäure, die anschließende Cyclisierung im basischen Milieu unter Erwärmung, beispielsweise in halbkonz. Natronlauge.

Eine gewünschtenfalls vorzunehmende Reduktion erfolgt zweckmäßig in einem Lösungsmittel wie beispielsweise einem niederen Alkohol oder Wasser oder einer Mischung derselben wie beispielsweise verd. Ethanol mit einem Reduktionsmittel wie Natriumdithionit. Sie kann jedoch auch in einem Lösungsmittel wie einem niederen Alkohol mit katalytisch erregtem Wasserstoff unter Nickelkatalyse oder in Essigsäure mit Eisenpulver oder Zink durchgeführt werden.

Eine gewünschtenfalls vorzunehmende Diazotierung erfolgt zweckmäßig mit salpetriger Säure, dargestellt aus Alkalinitriten wie z.B. Natriumnitrit in Säuren wie z.B. Essigsäure, Salzsäure oder Schwefelsäure bei Temperaturen zwischen -20 °C und +30 °C. Zur Hydrolyse kann in wässrigem Medium stehengelassen oder gewünschtenfalls erwärmt werden.

Als pharmakologisch verträgliche Salze kommen insbesondere Alkali-, Erdalkali- und Ammoniumsalze sowie gegebenenfalls Salze mit nichttoxischen anorganischen oder organischen Säuren, wie z.B. Salzsäure, Schwefelsäure, Phosphorsäure, Bromwasserstoffsäure, Essigsäure, Milchsäure, Zitronensäure, Äpfelsäure, Benzoesäure, Salicylsäure, Malonsäure, Maleinsäure, Bemsteinsäure oder Diaminocapronsäure in Frage.

Die Salze erhält man in üblicher Weise z.B. durch Neutralisation der Verbindungen der Formel I mit den entsprechenden Laugen oder Säuren.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I bzw. I a können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und / oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Polymere (wie Polyethylenglykole).

Für die orale Applikation geeignete Zubereitungen können gewünschtenfalls Geschmacks- und Süßstoffe enthalten. Für die äußerliche Anwendung können die erfindungsgemäßen Substanzen 1 auch in Form von Pudern und Salben verwendet werden. Sie werden dazu z.B. mit pulverförmigen, physiologisch verträglichen Verdünnungsmitteln bzw. üblichen Salbengrundlagen vermischt. Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Außer den in den Beispielen genannten Substanzen sind im Sinne der vorliegenden Erfindung die folgenden Verbindungen bevorzugt:
7-Isopropyl-1,2,4-benzotriazin-3-ol
7-t-Butyl-1,2,4-benzotriazin-3-ol
6,7-Dimethyl-1 ,2,4-benzotriazin-3-ol
7-Hydroxy-1 ,2,4-benzotriazin-3-ol
6-Chlor-1 ,2,4-benzotriazin-3-ol
7-Chlor-1 ,2,4-benzotriazin-3-ol
7-Methoxy-1 ,2,4-benzotriazin-3-ol
7-Carboxymethoxy-1,2,4-benzotriazin-3-ol

### Beispiel 1

### 1,2,4-Benzotriazin-3-amin-1-oxid

Eine Mischung aus 10 g 2-Nitroanilin und 20 g Cyanamid wird 5 min auf 100 °C erhitzt, abgekühlt, mit 25 ml konz. Salzsäure versetzt und vorsichtig auf 70 °C erwärmt. Nach Abklingen der heftigen Reaktion kühlt man auf Raumtemp., tropft 50 ml halbkonz. Natronlauge zu, erhitzt 30 min auf 100 °C, kühlt ab, filtriert und wäscht den Niederschlag mit heißem Eisessig. Es verbleiben 9.1 g Titelverbindung (78 % d. Th.) vom Schmp. 272-274 °C.

### Beispiel 2

In analoger Weise wie in Beispiel 1 beschrieben erhält man:

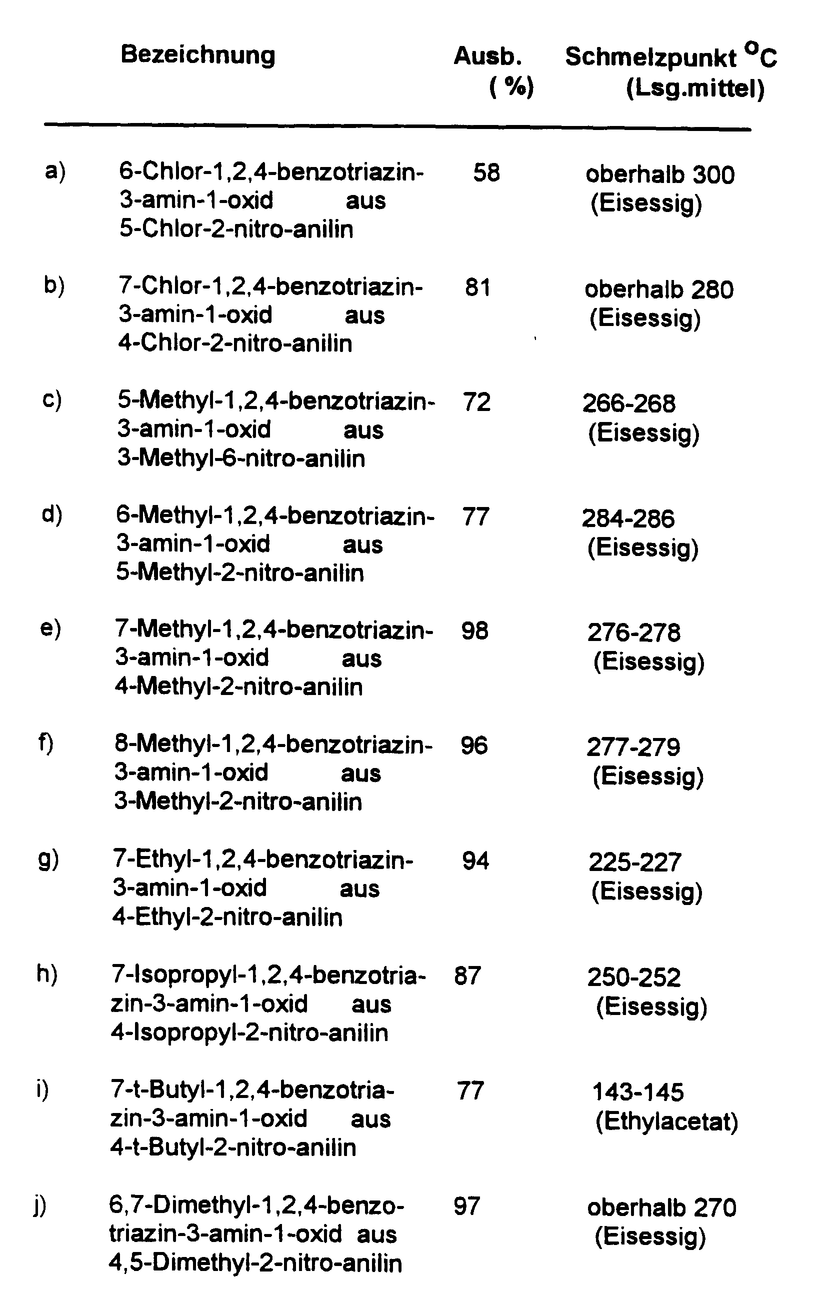

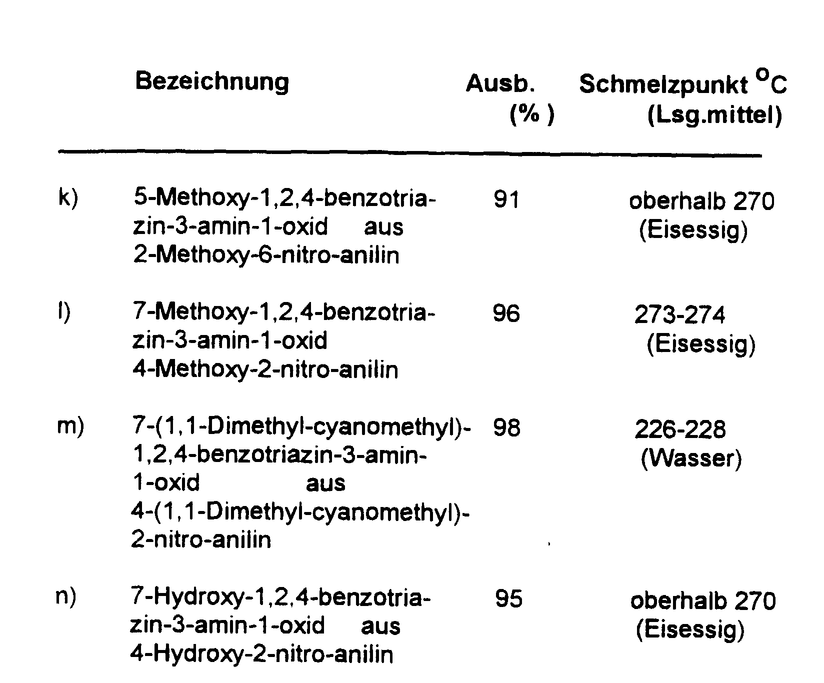

### Beispiel 3

### 1,2,4-Benzotriazin-3-amin

Eine Mischung aus 8.1 g (49 mmol) 1,2,4-Benzotriazin-3-amin-1-oxid, 8.1 g Natriumdithionit und 800 ml 70proz. Ethanol wird 1 h zum Rückfluß erhitzt. Man filtriert heiß, engt das Filtrat ein, wäscht den Rückstand mit Wasser und trocknet. Es ver bleiben 7.0 g 1,2,4-Benzotriazin-3-amin (98 % d. Th.) vom Schmp. 208-210 °C.

### Beispiel 4

In analoger Weise wie in Beispiel 3 beschrieben erhält man:

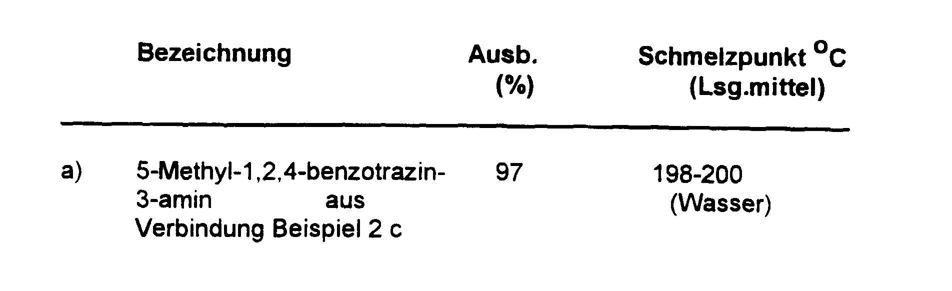

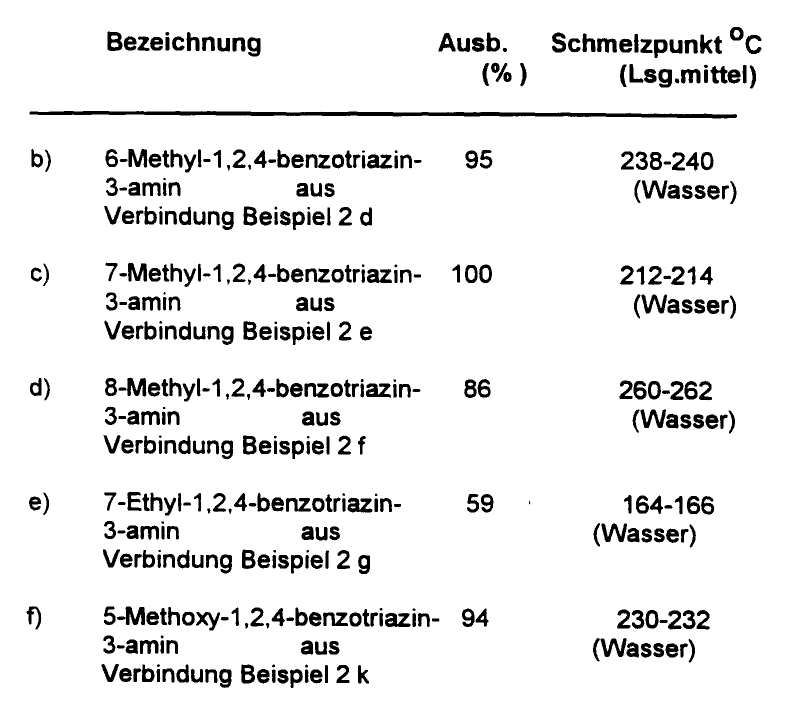

### Beispiel 5

### 1,2,4-Benzotriazin-3-ol

Zu einer Lösung von 2.0 g (14 mmol) 1,2,4-Benzotriazin-3-amin in 30 ml Wasser und 3 ml konz. Schwefelsäure tropft man bei 10 °C eine Lösung von 1 g Natriumnitrit in 10 ml Wasser. Man rührt 4 h bei Raumtemp. nach, filtriert den Niederschlag ab und trocknet. Man erhält 1.1 g Titelverbindung (55 % d. Th.) vom Schmp. 204-206 °C.

### Beispiel 6

In analoger Weise wie in Beispiel 5 beschrieben erhält man:

| | **Bezeichnung** | **Ausb. (%)** | **Schmelzpunkt °C (Lsg.mittel)** |
|---|---|---|---|
| a) | 5-Methyl-1,2,4-benzotriazin-3-ol aus Verbindung Beispiel 4 a | 95 | 206-208 (Wasser) |
| | | | |
| b) | 6-Methyl-1,2,4-benzotriazin-3-ol aus Verbindung Beispiel 4 b | 73 | oberhalb 300 (Ether) |
| | | | |
| c) | 7-Methyl-1,2,4-benzotriazin-3-ol aus Verbindung Beispiel 4 c | 54 | 202-204 (Ether) |
| | | | |
| d) | 8-Methyl-1,2,4-benzotriazin-3-ol aus Verbindung Beispiel 4 d | 62 | 195-197 (Ether) |
| | | | |
| e) | 7-Ethyl-1,2,4-benzotriazin-3-ol aus Verbindung Beispiel 4 e | 39 | 180-182 (Isohexan) |
| | | | |
| f) | 5-Methoxy-1,2,4-benzotriazin-3-ol aus Verbindung Beispiel 4 f | 57 | 186-188 (Ether) |

### Beispiel 7

### 1,2,4-Benzotriazin-3-ol-1-oxid

Zu einer Lösung von 7.5 g (46 mmol) 1,2,4-Benzotriazin-3-amin-1-oxid (Chem. Ber. 46, 3522 (1913)) in 75 ml Wasser und 27 ml konz. Schwefelsäure tropft man bei Raumtemp. eine Lösung von 13.3 g Natriumnitrit in 25 ml Wasser. Man läßt über Nacht stehen, filtriert, nimmt in Sodalösung auf, wäscht mit Ether, säuert die wässrige Phase an, filtriert und kristallisiert aus Eisessig um. Man isoliert 4.7 g Titelverbindung (63 % d. Th.) vom Schmp. 238-239 °C).

### Beispiel 8

In analoger Weise wie in Beispiel 7 beschrieben erhält man:

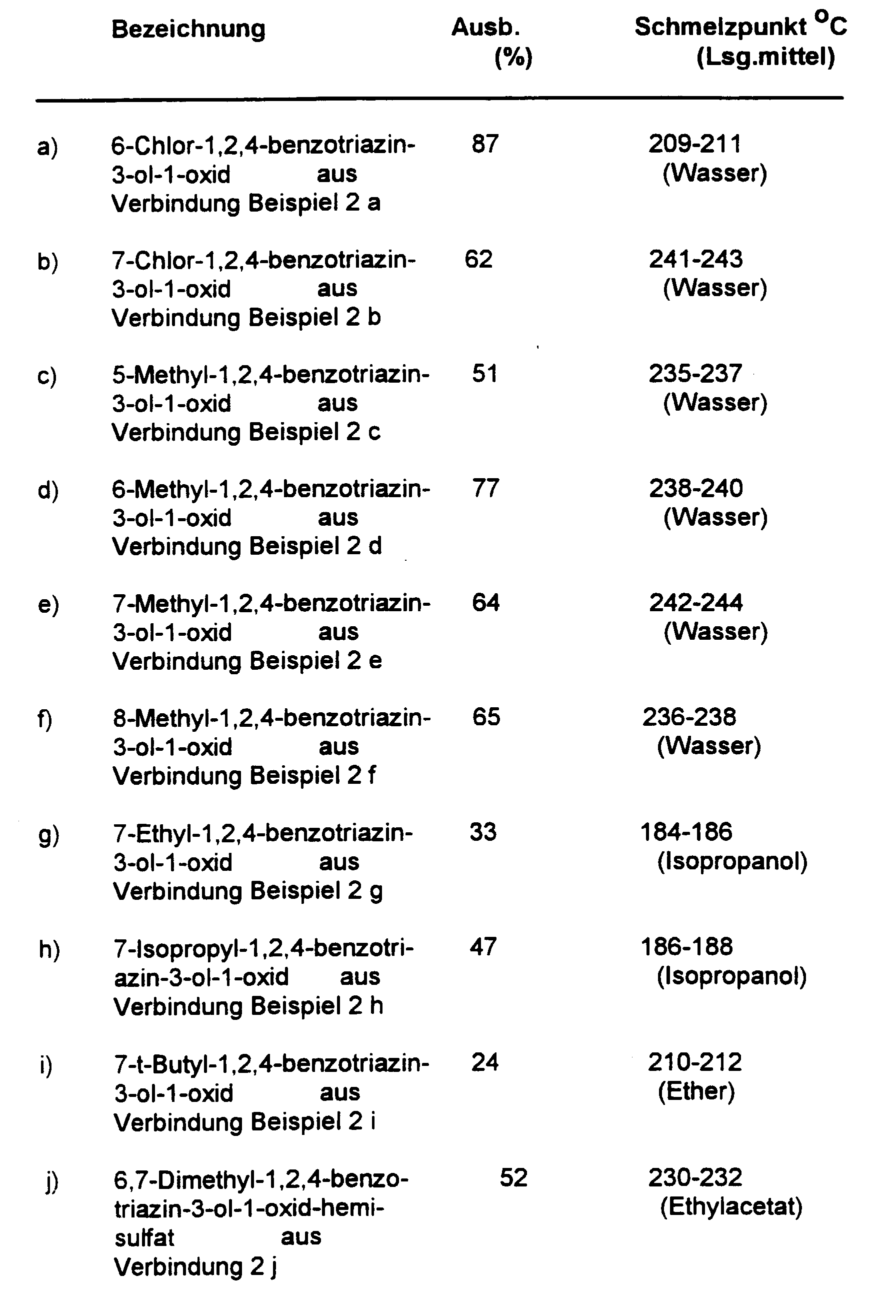

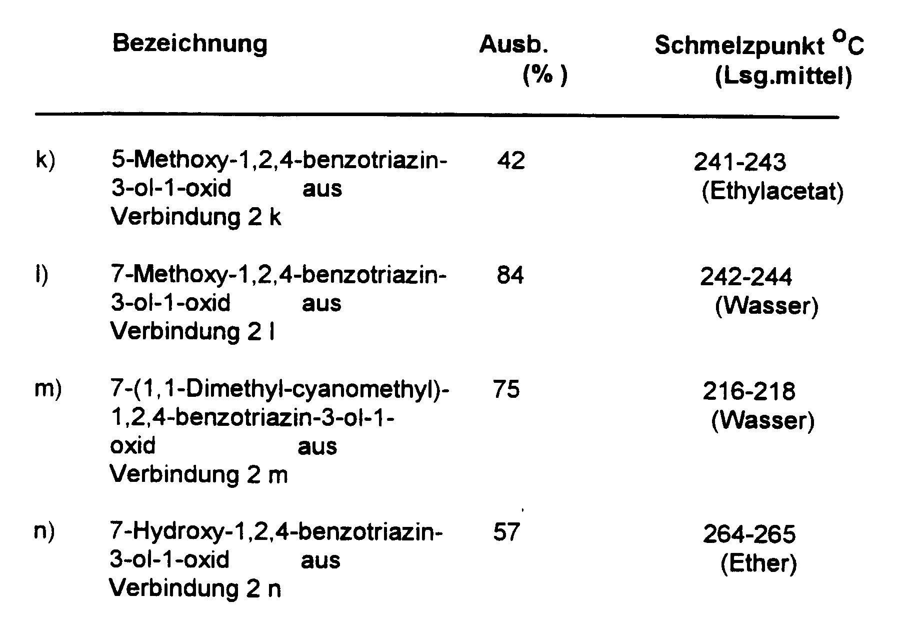

### Beispiel 9

### 7-(1,1-Dimethyl-cyanomethyl)-1,2,4-benzotriazin-3-ol

Eine Lösung von 1.5 g (6.5 mmol) Verbindung des Beispiels 8m in 30 ml Eisessig wird bei 85 °C innerhalb 30 min mit 1.8 g Eisenpulver versetzt. Man saugt heiß ab, wäscht mit heißem Eisessig nach, engt das Filtrat ein und chromatographiert an Kieselgel. Nach Elution mit Ethylacetat/Methanol 3:1 und Verreiben mit Ether verbleiben 0.8 g Titelverbindung (57 % d. Th.) vom Schmp. 183-185 °C.

### Beispiel 10

### Hemmung der PLA₂-Aktivität

a) Hemmung der humanen rekombinanten Typ-II-PLA₂ (= synoviale PLA₂)
Als typischer Repräsentant einer PLA₂ wurde die synoviale PLA₂ (Dissertation Rainer Müller, 1993; Universität Regensburg) zur Testung ausgewählt.
Als Vertreter der Verbindungen der Formel I bzw. I a wurde die Verbindung aus Beispiel 1 näher untersucht. In der folgenden Tabelle wird die prozentuale in-vitro Hemmung der PLA₂ dosisabhängig angegeben:

| **Verbindung aus Beispiel** | **PLA**_{**2**}**-Hemmung [%]** |
|---|---|
| | |
| 1 100 µg/ml | 99 |
| 1 10 µg/ml | 60 |
| 1 1 µg/ml | 43 |

b) Hemmung der collageninduzierten Arachidonsäure (AA)-Freisetzung aus humanen Thrombozyten

Als weiterer Indikator einer PLA₂-Inhibition gilt eine Hemmung der AA-Freisetzung aus Thrombozyten. Zu diesem Zweck werden die Thrombozyten mit ³H-AA äquilibriert. Diese zugegebene radioaktive Arachidonsäure baut sich in Membranphospholipide ein. Anschließend wird die thrombozytäre PLA₂ über den Collagenrezeptor aktiviert und ³H-AA so aus den Membranphospholipiden wieder ins Medium freigesetzt, wo sie gemessen werden kann.

In Tabelle 1 ist gezeigt, daß die repräsentativ ausgewählte Verbindung des Beispiels 1 die collageninduzierte AA-Freisetzung bis zu 91 % dosisabhängig hemmt.

| **Verbindung aus Beispiel** | **Hemmung der AA-Freisetzung [%]** |
|---|---|
| | |
| 1 100 µg/ml | 91 |
| 1 10 µg/ml | 29 |

## Patentansprüche

1. Verwendung von Verbindungen der Formel I 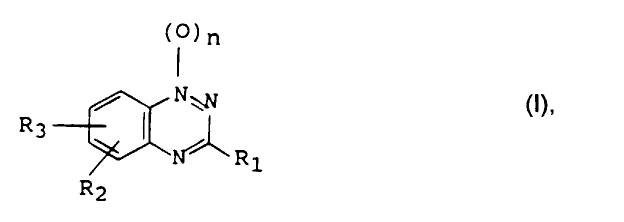 in welcher
R₁ eine Amino-, oder eine Hydroxygruppe,
R₂ und R₃, die gleich oder verschieden sind, jeweils Wasserstoff, Halogen, C₁-C₆-Alkyl, C₁-C₆-Alkoxy, Cyano, Carboxy, Cyano-C₁-C₆-alkyl, Carboxy-C₁C₆-alkyl, Hydroxy, Carboxy-C₁-C₆-alkoxy, C₁-C₆-Alkoxycarbonyl-C₁-C₆-alkoxy oder Aminocarbonyl-C₁-C₆-alkoxy, C₁-C₆-Alkylaminocarbonyl-C₁-C₆-alkoxy- oder Di-C₁-C₆-Alkylaminocarbonyl-C₁-C₆-alkoxy
und
n die Zahl 0 oder 1 bedeutet,
wobei Hal nicht Fluor sein darf
deren Tautomere sowie Salze mit nichttoxischen Säuren oder Basen,
zur Herstellung von Arzneimitteln mit PLA₂-inhibierender Wirkung.

2. Verwendung von Verbindungen der Formel I gemäß Anspruch 1 wobei R₂ ein Wasserstoff- oder Chlor, eine C₁-C₆-Alkyl-, C₁-C₆-Alkoxy-, Cyano-C₁-C₆-alkyl- oder Hydroxygruppe darstellt.

3. Verwendung von Verbindungen der Formel I gemäß Anspruch 1, oder 2, wobei R³ ein Wasserstoffatom oder eine C₁-C₆-Alkylgruppe bedeutet.

4. Verbindungen der Formel I a 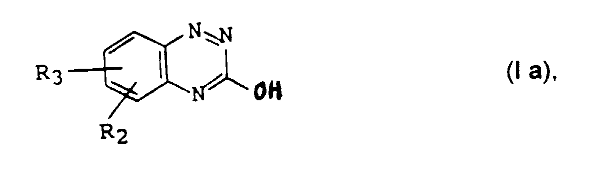 in der
R₂ C₁-C₆-Alkyl, C₁-C₆-Alkoxy, Cyano, Carboxy, Cyano-C₁-C₆-Alkyl, Carboxy-C₁-C₆-alkyl, Hydroxy, Carboxy-C₁-C₆-alkoxy, C₁-C₆-Alkoxycarbonyl-C₁-C₆-alkoxy, Aminocarbonyl-C₁-C₆-alkoxy, C₁-C₆-Alkylaminocarbonyl-C₁-C₆-alkoxy oder Di-C₁-C₆-Alkylaminocarbonyl-C₁-C₆-alkoxy und
R₃ Wasserstoff oder C₁-C₆-Alkyl bedeuten,
deren Tautomere sowie Salze mit nichttoxischen Säuren oder Basen.

5. Verbindungen der Formel I a gemäß Anspruch 4, wobei R₂ eine C₁-C₆-Alkyl-, C₁-C₆-Alkoxy-, Cyano-C₁-C₆-alkyl-, Hydroxy- oder Carboxy-C₁-C₆-alkoxygruppe bedeutet.

6. Verbindungen der Formel I a gemäß Anspruch 4 oder 5, wobei R₃ ein Wasserstoffatom oder eine C₁-C₆-Alkylgruppe bedeutet.

7. Verfahren zur Herstellung von Verbindungen der Formel I a gemäß Anspruch 4, 5 oder 6, dadurch gekennzeichnet, daß man eine Verbindung der Formel II 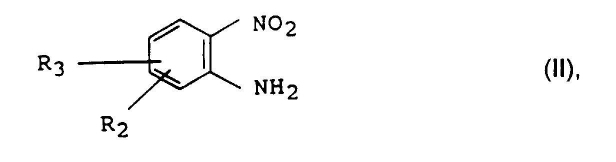
in welcher R₂ und R₃ die obengenannten Bedeutung haben,
mit Cyanamid umsetzt und unter basischen Bedingungen cyclisiert
und anschließend das erhaltene Produkt durch Reduktion und anschließende Diazotierung und Hydrolyse in eine Verbindung der Formel I,
in welcher R₂ und R₃ die obengenannte Bedeutung haben, überführt,
sowie gegebenenfalls durch Neutralisation mit nichttoxischen Säuren oder Basen in ein Salz umwandelt.

8. Arzneimittel, enthaltend eine Verbindung gemäß Anspruch 4, 5 oder 6 neben pharmakologisch verträglichen Träger- und Hilfsstoffen.

9. Arzneimittel gemäß Anspruch 8 zur Behandlung von entzündlichen Erkrankungen.

## Claims

1. Use of compounds of the formula I 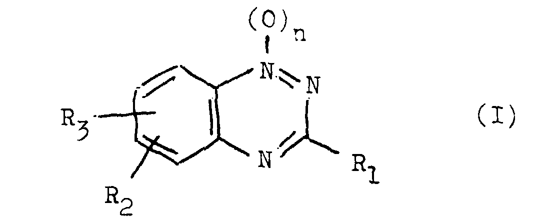 in which R₁ signifies an amino or a hydroxyl group, R₂ and R₃, which are the same or different, each hydrogen, halogen, C₁-C₆-alkyl, C₁-C₆-alkoxy, cyano, carboxyl, cyano-C₁-C₆-alkyl, carboxy-C₁-C₆-alkyl, hydroxyl, carboxy-C₁-C₆-alkoxy, C₁-C₆-alkoxycarbonyl-C₁-C₆-alkoxy or aminocarbonyl-C₁-C₆-alkoxy, C₁-C₆-alkylaminocarbonyl-C₁-C₆-alkoxy or di-C₁-C₆-alkylaminocarbonyl-C₁-C₆-alkoxy and n the number 0 or 1, whereby Hal may not be fluorine, their tautomers, as well as salts with non-toxic acids or bases, for the preparation of medicaments with PLA₂-inhibiting action.

2. Use of compounds of the formula I according to claim 1, whereby R₂ represents a hydrogen or chlorine atom, a C₁-C₆-alkyl, C₁-C₆-alkoxy, cyano-C₁-C₆-alkyl or hydroxyl group.

3. Use of compounds of the formula I according to claim 1 or 2, whereby R³ signifies a hydrogen atom or a C₁-C₆-alkyl group.

4. Compounds of the formula Ia 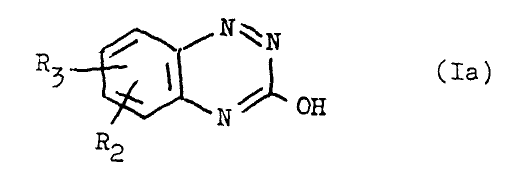 in which R₂ signifies C₁-C₆-alkyl, C₁-C₆-alkoxy, cyano, carboxyl, cyano-C₁-C₆-alkyl, carboxy-C₁-C₆-alkyl, hydroxyl, carboxy-C₁-C₆-alkoxy, C₁-C₆-alkoxycarbonyl-C₁-C6-alkoxy, aminocarbonyl-C₁-C₆-alkoxy, C₁-C₆-alkylaminocarbonyl-C₁-C₆-alkoxy or di-C₁-C₆-alkylaminocarbonyl-C₁-C₆-alkoxy and R₃ hydrogen or C₁-C₆-alkyl, their tautomers, as well as salts with non-toxic acids or bases.

5. Compounds of the formula Ia according to claim 4, whereby R₂ signifies a C₁-C₆-alkyl, C₁-C₆-alkoxy, cyano-C₁-C₆-alkyl, hydroxyl or carboxy-C₁-C₆-alkoxy group.

6. Compounds of the formula Ia according to claim 4 or 5, whereby R₃ signifies a hydrogen atom or a C₁-C₆-alkyl group.

7. Process for the preparation of compounds of the formula Ia according to claim 4, 5 or 6, characterised in that one reacts a compound of the formula II 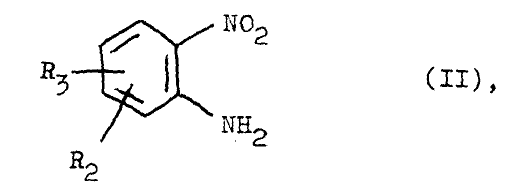 in which R₂ and R₃ have the above-mentioned meaning, with cyanamide and cyclises under basic conditions and subsequently converts the product obtained by reduction and subsequent diazotisation and hydrolysis into a compound of the formula I, in which R₂ and R₂ have the above-mentioned meaning, as well as possibly converts into a salt by neutralisation with non-toxic acids or bases.

8. Medicament containing a compound according to claim 4, 5 or 6, besides pharmacologically compatible carrier and adjuvant materials.

9. Medicament according to claim 8 for the treatment of inflammatory diseases.

## Revendications

1. Utilisation de composés de formule I 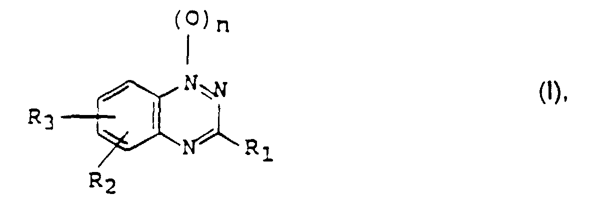 dans laquelle
R₁ représente un groupe amino ou un groupe hydroxy,
R₂ et R₃, qui sont identiques ou différents, représentent chacun un atome d'hydrogène, un atome d'halogène, un groupe alkyle en C₁-C₆, alcoxy en C₁-C₆, cyano, carboxy, cyano(alkyle en C₁-C₆), carboxy(alkyle en C₁-C₆), hydroxy, carboxy(alcoxy en C₁-C₆), (alcoxy en C₁-C₆)-carbonyl-(alcoxy en C₁-C₆) ou aminocarbonyl(alcoxy en C₁-C₆), (alkyle en C₁-C₆)aminocarbonyl(alcoxy en C₁-C₆) ou di-(alkyle en C₁-C₆)aminocarbonyl(alcoxy en C₁-C₆)
et
n vaut 0 ou 1,
où Hal ne doit pas représenter un atome de fluor,
leurs tautomères ainsi que leurs sels formés avec des acides ou bases non toxiques,
pour la préparation de médicaments ayant une activité inhibitrice de PLA₂.

2. Utilisation de composés de formule I selon la revendication 1, où R₂ représente un atome d'hydrogène ou de chlore, un groupe alkyle en C₁-C₆, alcoxy en C₁-C₆, cyano(alkyle en C₁-C₆) ou hydroxy.

3. Utilisation de composés de formule I selon la revendication 1 ou 2, où R³ représente un atome d'hydrogène ou un groupe alkyle en C₁-C₆.

4. Composés de formule la 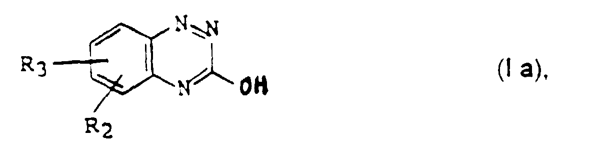 dans laquelle
R₂ représente un groupe alkyle en C₁-C₆, alcoxy en C₁-C₆, cyano, carboxy, cyano(alkyle en C₁-C₆), carboxy(alkyle en C₁-C₆), hydroxy, carboxy(alcoxy en C₁-C₆), (alcoxy en C₁-C₆)-carbonyl-(alcoxy en C₁-C₆), aminocarbonyl(alcoxy en C₁-C₆), (alkyle en C₁-C₆)aminocarbonyl(alcoxy en C₁-C₆) ou di-(alkyle en C₁-C₆)aminocarbonyl(alcoxy en C₁-C₆) et
R₃ représente un atome d'hydrogène ou un groupe alkyle en C₁-C₆.
leurs tautomères ainsi que leurs sels formés avec des acides ou bases non toxiques.

5. Composés de formule I selon la revendication 4, où R₂ représente un groupe alkyle en C₁-C₆, alcoxy en C₁-C₆, cyano(alkyle en C₁-C₆, hydroxy ou carboxy(alcoxy en C₁-C₆).

6. Composés de formule I selon la revendication 4 ou 5, où R₃ représente un atome d'hydrogène ou un groupe alkyle en C₁-C₆.

7. Procédé de préparation de composés de formule I selon la revendication 4, 5 ou 6, caractérisé en ce que l'on fait réagir un composé de formule II 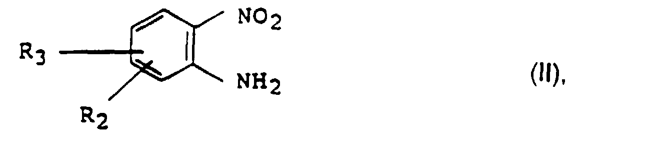 dans laquelle R₂ et R₃ ont les significations mentionnées ci-dessus, avec du cyanamide et on le cyclise dans des conditions basiques, et ensuite, on transforme le produit obtenu, par réduction ainsi que diazotation et hydrolyse consécutives, en un composé de formule I
dans laquelle R₂ et R₃ ont les significations mentionnées ci-dessus, que l'on transforme éventuellement en un sel par neutralisation avec des acides ou bases non toxiques.

8. Médicament, contenant un composé selon la revendication 4, 5 ou 6, en plus de véhicules et adjuvants pharmacologiquement acceptables.

9. Médicament selon la revendication 8, destiné au traitement de maladies inflammatoires.
